(19) Europäisches Patentamt
European Patent Office
Office européen des brevets

(11) Veröffentlichungsnummer: **0 568 004 A2**

(12) **EUROPÄISCHE PATENTANMELDUNG**

(21) Anmeldenummer: **93106804.3**

(22) Anmeldetag: **27.04.93**

(51) Int. Cl.[5]: **C12P 35/00**, C12P 35/06, C12P 35/02, C12N 1/20, C12N 9/84, //(C12P35/00, C12R1:01),(C12P35/06, C12R1:01),(C12N1/20, C12R1:01),(C12P35/02, C12R1:38),(C12N9/84, C12R1:38)

Die Mikroorganismen sind bei DSM Deutsche Sammlung von Mikroorganismen und Zellkulturen GmbH unter den Nummern DSM 7007 und DSM 7509 hinterlegt worden.

(30) Priorität: **29.04.92 CH 1381/92**

(43) Veröffentlichungstag der Anmeldung: **03.11.93 Patentblatt 93/44**

(84) Benannte Vertragsstaaten: **AT BE CH DE DK ES FR GB GR IE IT LI NL PT SE**

(71) Anmelder: **LONZA AG**
**Gampel/Wallis**
**Geschäftsleitung Basel**
**CH-4002 Basel(CH)**

(72) Erfinder: **Schurr, Sabine**
**Blauenstrasse 24**
**W-Eimeldingen(DE)**
Erfinder: **Tschech, Andreas, Dr.**
**Ziegelrain 23**
**CH-Aarau (Kanton Aargau)(CH)**

(74) Vertreter: **Weinhold, Peter, Dr. et al**
**Patentanwälte Dipl.-Ing. G. Dannenberg**
**Dr. P. Weinhold**
**Dr. D. Gudel**
**Dipl.-Ing. S. Schubert**
**Dr. P. Barz**
**Siegfriedstrasse 8**
**D-80803 München (DE)**

(54) **Mikorbioologisches Verfahren zur Herstellung von Malonyl-7-Aminocephalosporansäure-Derivaten.**

(57) Beschrieben werden neue Mikroorganismen, die derart selektioiniert sind, dass sie befähigt sind, das Lacton oder dessen lösliche Salze der Formel

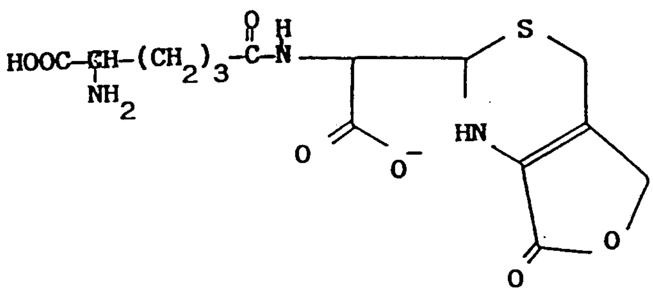

I

EP 0 568 004 A2

als einzige Kohlenstoff-, Stickstoff- und Energiequelle über Malonyl-Lacton oder dessen lösliche Salze der Formel

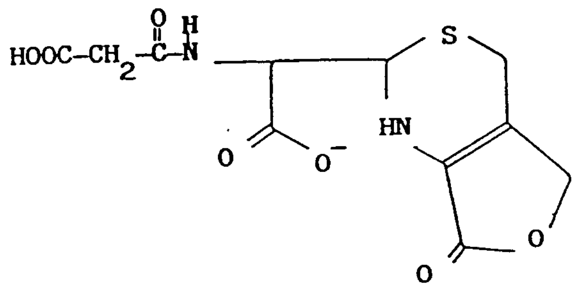

II

zu verwerten und letzteres nicht zu katabolisieren. Des weiteren wird wird ein mikrobiologisches Verfahren zur Herstellung von Malonyl-7-Aminocephalosporansäure-Derivaten oder deren lösliche Salze der allgemeinen Formel

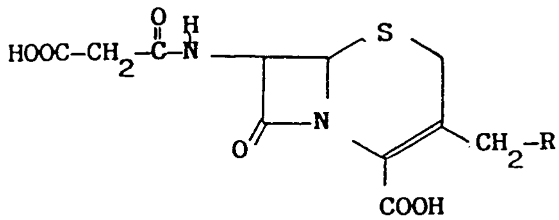

III

mit diesen Mikroorganismen ausgehend von Cephalosporin-C-Derivaten oder deren lösliche Salze der allgemeinen Formel

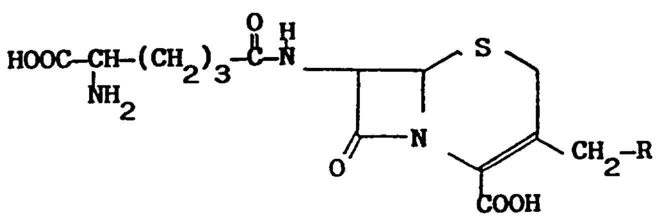

IV

beschrieben.
Des weiteren wird ausgehend von den Malonyl-7-Aminocephalosporansäure-Derivaten ein neues Verfahren zur Herstellung von 7-Aminocephalsporansäure-Derivaten der allgemeinen Formel

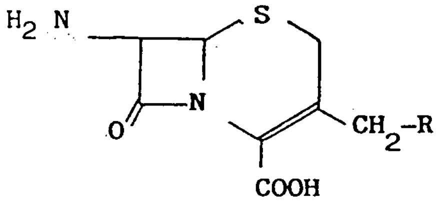

V

beschrieben.

Die Erfindung betrifft neue Malonyl-7-Aminocephalosporansäure-Derivate der allgemeinen Formel

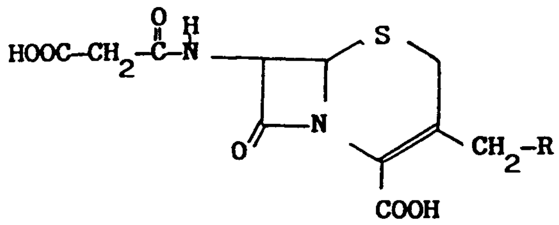

III

oder deren lösliche Salze,
worin R ein Wasserstoffatom eine Hydroxylgruppe oder eine Acetoxygruppe bedeutet, ein neues Verfahren zu deren Herstellung ausgehend von den entsprechenden Cephalosporin C-Derivaten sowie neue für das Verfahren geeignete Mikroorganismen.
Des weiteren betrifft die Erfindung ein neues mikrobiologisches Verfahren zur Herstellung von 7-Aminocephalosporansäure-Derivaten ausgehend von Malonyl-7-Aminocephalosporansäure-Derivaten sowie die neue Verwendung von Mikroorganismen der Gattung Pseudomanas sp. (DSM 7509).

Im folgenden werden als Malonyl-7-Aminocephalosporansäure-Derivate (Formel III), als Cephalosporin C-Derivate (Formel IV), als Malonyl-Lacton gemäss Formel II und als Lacton gemäss Formel I auch deren lösliche Salze, wie beispielsweise deren Ammonium- oder Alkalisalze verstanden.

Malonyl-7-Aminocephalosporansäure-Derivate können beispielsweise als Ausgangsmaterial zur Herstellung von 7-Aminocephalosporansäure-Derivaten dienen, welche wiederum wichtige Ausgangsverbindungen zur Herstellung von Cephalosporin-Antibiotika sind (J. Bacteriol., 1987, Vol. 169, Nr. 12, S. 5815-5820).

Bisher sind weder chemische noch mikrobiologische Verfahren zur Herstellung von Malonyl-7-Aminocephalosporansäure-Derivaten bekannt.
Zur Herstellung von 7-Aminocephalosporansäure sind mehrere mikrobiologische Verfahren ausgehend von Cephalosporin C bekannt wie bspw. das in der JP-A 62 48380 beschriebene. Diese Verfahren haben jedoch alle den Nachteil, dass sie grosstechnisch nicht gangbar sind.

Aufgabe der Erfindung war die Bereitstellung von neuen Malonyl-7-Aminocephalosporansäure-Derivaten. Eine weitere Aufgabe der Erfindung war, mit neuen Mikroorganismen ein neues, einfaches und wirtschaftliches mikrobiologisches Verfahren zur Herstellung von neuen Malonyl-7-Aminocephalosporansäure-Derivaten zur Verfügung zu stellen. Eine weitere Aufgabe war, mit diesen neuen Derivaten ein neues mikrobiologisches Verfahren zur Herstellung von 7-Aminocephalosporansäure-Derivaten zur Verfügung zu stellen.

Erfindungsgemäss werden diese Aufgaben mit den neuen Mikroorganismen gemäss Patentanspruch 1, mit dem neuen Verfahren gemäss Patentanspruch 3, mit den neuen Malonyl-7-Aminocephalosporansäure-Derivaten gemäss Patentanspruch 8 und mit dem neuen Verfahren gemäss Patentanspruch 10 gelöst.

Die erfindungsgemässen Mikroorganismen sind derart selektioniert, dass sie befähigt sind, das Lacton der Formel

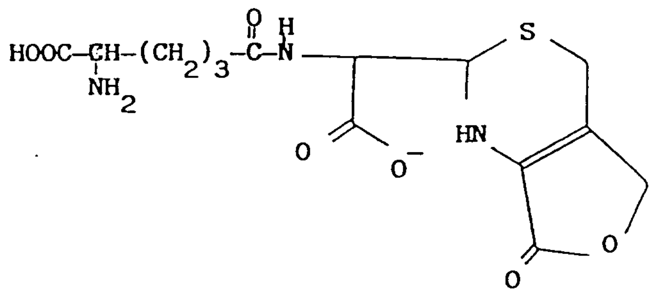

I

als einzige Kohlenstoff-, Stickstoff- und Energiequelle über Malonyl-Lacton der Formel

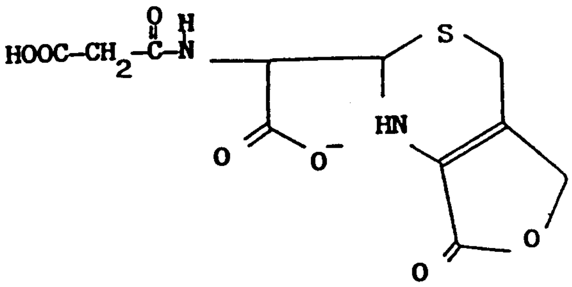

II

zu verwerten und letzteres nicht zu katabolisieren.

Diese Mikroorganismen werden mit dem Lacton gemäss Formel I unter Zuhilfenahme traditioneller mikrobiologischer Techniken, beispielsweise aus diversen Erdproben, selektioniert. D.h. züchtet man aus Erdproben als Inokulum mit dem Lacton gemäss Formel I Mikroorganismen an, erhält man Mikroorgansmen, die befähigt sind, mit diesem als einzige Kohlenstoff-, Stickstoff- und Energiequelle zu wachsen. Aus diesen Mikroorganismen werden dann nach fachmännisch üblichen Methoden die selektioniert, die das Lacton gemäss Formel I in Malonyl-Lacton gemäss Formel II überführen und die letzteres nicht katabolisieren.

Das für die Selektion notwendige Lacton, Formel I, kann aus käuflichem Desacetylcephalosporin C erhalten werden. Hierzu wird zunächst auf bekannte Weise (Jefferay et al., Biochem. J., 1961, 81, S. 591) Desacetylcephalosporin C zum entsprechenden Lacton lactonisiert. Zur Bildung des gewünschen Lactons mit gespaltenem Lactam-Ring gemäss Formel I wird dann der Lactam-Ring fachmännisch beispielsweise mittels Penicillinase gespalten.

Prinzipiell sind alle Mikroorganismen geeignet, die unter diesem Selektionsdruck erhalten werden. Diese Mikroorganismen sind in der Literatur nicht beschrieben und Bestandteil der Erfindung.
Zweckmässig werden durch die Selektion die Mikroorganismen mit der Bezeichnung FB1 (DSM 7007) sowie deren Mutanten und Deszendenten erhalten. Diese sind am 25.3.1992 bei der Deutschen Sammlung für Mikroorganismen und Zellkulturen GmbH, Mascheroderweg 1b, D-3300 Braunschweig, hinterlegt worden.

Diese Mikroorganismen sind nach dem Prioritätstag dieser Anmeldung über 16 S RNA-Analyse zu Sphingomonas sp. gehörend identifiziert worden.

Wissenschaftliche Beschreibung von Sphingomonas sp. FB1(DSM 7007):

| Zellform | winzige Stäbchen |
|---|---|
| Breite µm | 0.4-0.6 |
| Länge µm | 0.8-1.5 |
| Beweglichkeit | + |
| Geisseln | polar 1 |
| Gram-Reaktion | - |
| Lyse durch 3% KOH | + |
| Aminopeptidas (Cerny) | + |
| Sporen | - |
| Oxidase | + |
| Catalase | + |

Hauptchinonkomponente: Ubichinon Q10
DNA-Basenzusammensetzung nach HPLC-Schnellmethode: 63 mol % G+C.

Üblicherweise erfolgt die Selektion und die Anzucht in einem Mineralsalzmedium, zweckmässig in einem Mineralsalzmedium, dessen Zusammensetzung in Tabelle I (a und b) angegeben ist. Zur Anzucht der Mikroorganismen nach erfolgter Selektion sind jedoch auch andere Medien, wie beispielsweise handelsübliche Vollmedien, denkbar.

Zweckmässig wird zur Anzucht und Selektion das Lacton gemäss Formel I in einer Menge von 0,2 bis 1 Gew%, vorzugsweise von 0,3 bis 0,6 Gew.%, dem Mineralsalzmedium hinzugefügt.

Die Temperatur liegt zweckmässig während der Selektion und der Anzucht zwischen 0 und 60 °C, vorzugsweise zwischen 20 und 40 °C.

Selektion und Anzucht werden zweckmässig bei einem pH von 5 bis 9, vorzugsweise von 6 bis 8 durchgeführt.

Ist eine zweckmässige optische Dichte bei 650 nm ($OD_{650}$) von 0,1 bis 1 erreicht, können die Mikroorganismen nach fachmännisch üblichen Methoden geerntet und für das erfindungsgemässe Verfahren eingesetzt werden.

Erfindungsgemäss werden die auf diese Weise selektionierten Mikroorganismen im erfindungsgemässen Verfahren für die Umsetzung von Cephalosporin C-Derivaten der allgemeinen Formel

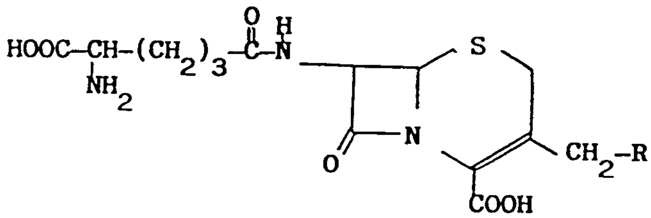

IV

worin R ein Wasserstoffatom, eine Hydroxylgruppe oder eine Acetoxygruppe bedeutet, als Substrat, zu Malonyl-7-Aminocephalosporansäure-Derivaten der allgemeinen Formel

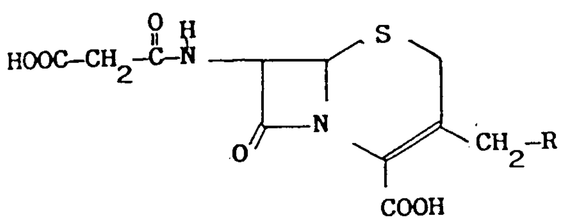

III

worin R die genannte Bedeutung hat, eingesetzt.

Zweckmässig wird das Verfahren mit den selektionierten Mikroorganismen Sphingomonas sp. FB1 (DSM 7007) oder deren Deszendenten und Mutanten durchgeführt, die wie bereits beschrieben hinterlegt sind.

Üblicherweise wird das Verfahren auf fachmännisch übliche Weise mit nicht wachsenden Zellen durchgeführt.

Als Substrate dienen Cephalosporin C-Derivate der allgemeinen Formel IV, worin R die genannte Bedeutung hat. Zweckmässig dient als Substrat Cephalosporin C (R in der allgemeinen Formel III = eine Acetoxygruppe).

Das Substrat kann für das Verfahren einmalig oder kontinuierlich zugegeben werden. Zweckmässig erfolgt die Substratzugabe so, dass die Substratkonzentration im Kulturmedium 20 Gew%, vorzugsweise 4 Gew%, nicht übersteigt.

Als Medien können für das Verfahren fachmännisch übliche angewendet werden. Vorzugsweise wird das Verfahren In niedermolarem HEPES-Puffer (4-(2-Hydroxyethyl)-piperazin-1-ethan-sulfonsäure) durchgeführt.

Üblicherweise wird das Verfahren mit einer Mikroorganismen-Suspension durchgeführt, die eine $OD_{650}$ von 1 bis 100, vorzugsweise von 2 bis 50 aufweist.

Das Verfahren wird zweckmässig bei einer Temperatur von 0 bis 60° C, vorzugsweise von 20 bis 40°C und bei einem pH-Wert von 5 bis 9, vorzugsweise von 6 bis 8, durchgeführt.

Nach einer üblichen Umsetzungsdauer von 1 bis 24 h können dann die bisher nicht beschriebenen Malonyl-7-Aminocephalosporansäure-Derivate gemäss Formel III auf fachmännisch übliche Weise isoliert werden. Als bevorzugter Vertreter der Malonyl-7-Aminocephalosporansäure-Derivate wird Malonyl-7-Aminocephalosporansäure (R in der allgemeinen Formel III = eine Acetoxygruppe) isoliert.

Das erfindungsgemässe Verfahren zur Herstellung von 7-Aminocephalosporansäure-Derivaten der allgemeinen Formel

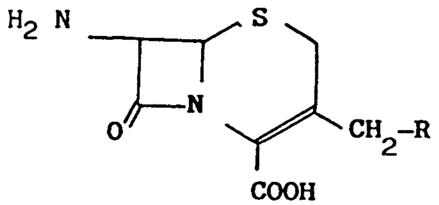

V

oder deren lösliche Salze,
worin R die genannte Bedeutung hat, wird derart durchgeführt, dass man Malonyl-7-Aminocephalosporansäure-Derivate oder deren lösliche Salze der allgemeinen Formel III, als Substrat, mittels Mikroorganismen der Gattung Pseudomonas sp (DSM 7509) oder deren Deszendenten und Mutanten oder mit zellfreien Enzymen aus diesen Mikroorganismen, in das Produkt gemäss Formel V überführt.

Die Mikroorganismen Pseudomonas sp (DSM 7509) sind am 5.3.1993 bei der Deutschen Sammlung für Mikroorganismen und Zellkulturen GmbH, Mascheroderweg 1b, D-3300 Braunschweig, hinterlegt worden.

Diese Mikroorganismen sind bekannt und in der JP-A 62 483 80 als Mikroorganismen Pseudomonas sp. SE-495 (FERM BP-818) beschrieben. Gemäss der JP-A 62 483 80 sind diese Mikroorganismen derart selektioniert, dass sie Glutaryl-7-Aminocephalosporansäure zu 7-Aminocephalosporansäure hydrolysieren. Nicht beschrieben ist die Verwendung dieser Mikroorganismen zur Hydrolyse von Malonyl-7-Aminocephalosporansäure.

Demnach betrifft die vorliegende Erfindung auch der Verwendung dieser Mikroorganismen zur Hydrolyse von Malonyl-7-Aminocephalosporansäure-Derivaten (Formel V), vorzugsweise von Malonyl-7-Aminocephalosporansäure (R = Acetoxy-).

Zweckmässig wird dieses Verfahren entweder mit permeabilisierten Mikroorganismenzellen oder mit einem zellfreien Enzymextrakt durchgeführt, insbesondere mit einem zellfreien Enzymextrakt.

Zur Herstellung eines zellfreien Enzymextraktes können fachmännisch übliche Methoden wie beispielsweise die Behandlung mit Ultraschall oder mit "French-press" Anwendung finden.

Als Substrat dienen Malonyl-7-Aminocephalosporansäure-Derivate der allgemeinen Formel III, worin R die genannte Bedeutung hat. Zweckmässig dient als Substrat Malonyl-7-Aminocephalosporansäure (R = Acetoxy-).

Das Substrat kann einmalig oder kontinuierlich zugegeben werden, zweckmässig derart, dass die Substratkonzentration im Kulturmedium 10 Gew.%, vorzugsweise 2 Gew.% nicht übersteigt.

Als Medien können für dieses Verfahren fachmännisch übliche Nährmedien angewendet werden.

Wird das Verfahren mit einem zellfreien Enzymextrakt durchgeführt, weist die Proteinkonzentration dieses Extraktes zweckmässig 0,1 bis 20g pro l, vorzugsweise 1 bis 5g pro l, auf.

Zweckmässig wird das Verfahren bei einer Temperatur von 4 bis 50 °C, vorzugsweise von 20 bis 35 °C und bei einem pH-Wert von 4 bis 9, vorzugsweise von 7 bis 8, durchgeführt.

Nach einer üblichen Umsetzungsdauer von 3 bis 24 h können dann die 7-Aminocephalosporansäure-Derivate gemäss Formel V isoliert werden.

Malonyl-7-Aminocephalosporansäure-Derivate haben ebenso wie 7-Aminocephalosporansäure-Derivate antibiotische Wirkung.

Beispiel 1:

Isolation der Mikroorganismen

Zu 100 ml Mineralsalzmedium (Tabelle I), pH 7,0, enthaltend 5 mmolar (0,5 mmol/100 ml) das Lacton mit gespaltenem Lactam-Ring (Formel I) wurden diverse Erdproben aus der Umgebung von Visp (Schweiz) als Inokulum hinzugefügt und bei 25 ° C auf dem Schüttler bei 140 Upm (Umdrehungen pro Minute) inkubiert. Diese Kulturen wurden 4mal auf frisches Mineralsalzmedium überimpft, und anschliessend wurden sie auf diese Lacton-Umsetzung mittels analytischer HPLC überprüft.

Diese Kulturen wurden dann auf Mineralsalzmedium-Agar-Platten enthaltend 5 mmolar des Lactons (Formel I) bei 25 ° C inkubiert. Anschliessend wurde der Bakterienstamm Sphingomonas sp. FB1 (DSM 7007), der während des Wachstums aus diesem Lacton das Malonyl-Lacton der Formel II bildete und letzteres nicht vollständig abbaute, isoliert.

Beispiel 2:

Bildung von Malonyl-7-Aminocephalosporansäure (Formel III)

Die Mikroorganismen Sphingomonas sp. FB1 (DSM 7007) wurden in Mineralsalzmedium, pH 7,0 (Tabelle Ib) enthaltend 5 mmolar Lacton (Formel I) bis zu einer von $OD_{650}$ = 0,44 innerhalb von 4 Tagen angezüchtet. (Nach den ersten 2 Tagen wurde nochmals dieses Lacton zugegeben, so dass die Gesamtmenge 1 mmol Lacton pro 100 ml Medium betrug). Anschliessend wurden die Zellen abzentrifugiert und das Zell-Pellet in 20 ml 10 mmolarem HEPES-Puffer, pH 7,0, resuspendiert, wobei eine $OD_{650}$ von 2,2 eingestellt wurde. Zu dieser Zellsuspension wurden 0,4 mmol Cephalosporin C (Formel IV) hinzugefügt. Die Inkubation erfolgte auf dem Schüttler (140 Upm) bei 25 °C.

Nach 30 min, 60 min, 2,25 h, 5,0h und 24h wurde jeweils eine Probe mittels HPLC-Analyse untersucht. Die Bildungsrate an Malonyl-7-Aminocephalosporansäure betrug bei 2,25 h: 410 mg/1/h/$OD_{650}$.
Nach 5 h wurden 142 mg Malonyl-7-Aminocephalosporansäure analytisch nachgewiesen, was einer 99%igen Umsetzung von Cephalosporin C entsprach. Die Malonyl-7-Aminocephalosporansäure konnte mit einer Ausbeute von 40% mittels präparativer HPLC isoliert werden.

Beispiel 3:

Identifizierung von Malonyl-7-Aminocephalosporansäure (Formel III)

Zur Identifizierung des Produktes (Formel III) wurden 500 ml Mineralsalzmedium (Tabelle I), pH 7,0, mit einer Lösung von Lacton der Formel I versetzt, so dass die Endkonzentration 5 mmolar betrug. Dann wurde diese Lösung mit einer Vorkultur, deren $OD_{650}$ = 0,5 war, bei 25 °C und 140 Upm inokuliert. Nach einem Tag wurden nochmals 5 mmole Lacton (Formel I) pro 500 ml Medium hinzugegeben.
Als eine $OD_{650}$ von 0,5 erreicht war, wurden die Zellen abgeerntet. Dann wurde das Zell-Pellet in 30 ml 10 mmolarem HEPES-Puffer, pH 7,0, enthaltend 0,6 mmol Cephalosporin C (Formel IV) resuspendiert. Nach einer 5-stündigen Inkubation bei 25° C, bei 140 Upm, wurden die Zellen abzentrifugiert und aus dem Überstand die Malonyl-7-Aminocephalosporansäure mittels präparativer HPLC gereinigt. Die gereinigten Proben wurden über Nacht lyophilisiert und das Lyophilisat mittels NMR ($^{1}H$ und $^{13}C$) analysiert.
Durch Vergleich mit einer chemisch hergestellten Referenzsubstanz (hergestellt aus 7-Aminocephalosporansäure gemäss Bull. Soc. Chem. Belg., 1977, 86, S. 991 - 1002) wurde die Bildung von Malonyl-7-Aminocephalosporansäure bewiesen.

Malonyl-7-Aminocephalosporansäure Natrium-salz (chemischer Standard) $^{13}C$-NMR (DMSO, 100,5 MHz, $\delta$ in ppm):
20, s; 25, s; 40, m; 58, d; 64, S; 112, s; 134, s; 164, d; 170, t.
Malonyl-7-Aminocephalosporansäure (erfindungsgemäss)
13C-NMR (DMSO, 100,5 MHz, $\delta$ in ppm):
20, s; 25, s; 40, M; 58, d; 64, s; 112,s; 134, s; 164, d; 170, t.
$^{1}H$-NMR (chem. Standard, DMSO, 400 MHz, $\delta$ in ppm):
20, s; 2,5, s; 3,0, t; 3,5, m; 4,8, d; 5,0,d; 5,6 d.
$^{1}H$-NMR (erfindungsgemäss):
2,0,s; 2,5, s; 3,0, t; 3,5, m; 4,8, d; 5,0, d; 5,6, d.

Beispiel 4:

Lactonisierung von Desacetylcephalosporin

Ausgehend von Desacetylcephalosporin C (Ciba-Geigy AG, Basel) wurde Desacetylcephalosporin C-Lacton folgendermassen hergestellt:
100 g Desacetylcephalosporin C wurden in 1 l 20 mmolarem Natriumphosphatpuffer, pH 7,0, gelöst. Zu dieser Lösung wurde ein Ionenaustauscher (DOWEX 50X8 (Fluka) ($H^{+}$-Form)) zugegeben, bis der pH 2,5 erreichte. Nach 10 min Rühren (250 Upm, Raumtemperatur) wurde das Ionenaustauscherharz abfiltriert und der pH der Lösung mit konz. Salzsäure auf 0,8 eingestellt. Nach 1 h Rühren (Raumtemperatur, 250 Upm) wurde zur Reaktionslösung DOWEX 1X8 (Acetat-Form) (Fluka) zugegeben, bis ein pH von 3,0 - 3,2 erreicht war.

Dann wurde bei Raumtemperatur für weitere 1,5h bei 250 Upm gerührt und anschliessend das Ionenaustauscher-Harz abfiltriert. Dann wurde erneut DOWEX 1x8 (Acetat-Form) hinzugegeben, bis ein pH

von 3,3 - 3,5 erreicht war, 1 h gerührt (Raumtemperatur, 250 Upm) und anschliessend das Ionenaustauscher-Harz abfiltriert. Die Reaktionslösung wurde anschliessend am Rotavapor bis zur Trockene eingedampft (30 mbar, 30° C). Um die noch erhaltene Essigsäure zu entfernen, wurde das Produkt nach dem Eindampfen in Wasser gelöst, bis eine 35%ige Lösung erreicht war und dann der pH mit konz. Salzsäure auf 1,5 eingestellt.
Die so erhaltene Lösung wurde 2mal mit Ethylacetat (gleiches Volumen wie die Lösung) extrahiert. Die wässrigen Phasen wurden vereinigt und mit 3 molarer KOH-Lösung auf pH 3,5 eingestellt und am Rotavapor (30 mbar, 30°C) eingedampft. Das Produkt wurde anschliessend noch im Trockenschrank unter Vakuum (30 mbar, 20° C) vollständig getrocknet.
Die Gesamtausbeute betrug 60 g entsprechend einer Ausbeute von 60% ausgehend von 100 g Desacetylcephalosporin C.

Beispiel 5:

Herstellung des Lactons mit gespaltenem Lactam-Ring (Formel I)

Zur Herstellung von Lacton (Formel I) wurde Desacetylcephalosporin C-Lacton in Stocklösung 6 (Tabelle Ia), mit einer β-Lactamase, Penicillinase E.C. 3.5.2.6. (Sigma) behandelt. Die Reaktion startete unmittelbar nach Zugabe der Penicillinase.
Während der Reaktion wurde der pH-Wert durch Zugabe einer 1 normalen NaOH-Lösung auf pH 7,0 gehalten. Die Reaktion war beendet, sobald ein konstanter pH-Wert von 7,0 ohne NaOH-Zugabe, erreicht war. Nach Beendigung der Reaktion (ca. 2 h) wurde diese Lösung sterilfiltriert (0,2 µm Filter) und dann bei -80° C gelagert.

Beispiel 6:

Bildung von 7-Aminocephalosporansäure (Formel V)

Die Mikroorganismen Pseudomonas sp SE-495 (DSM 7509; FERM BP-818) wurden in einem Nährmedium (pH 7,0) enthaltend 0,2% w/v Fleischextrakt, 0,2% w/v Hefeextrakt, 0,5% w/v Pepton, 0,5% w/v Natriumglutamat und 0,005% w/v Magnesiumsulfat, bei 30°C, über Nacht angezüchtet. Diese Kulturen wurden in frisches Nährmedium der gleichen Zusammensetzung überimpft (Impfmenge: 10%) und 2 bis 4 Tage inkubiert.
Anschliessend wurden die Zellen durch Zentrifugation (20 min. bei 600 upm) abgeerntet, in 0,1 molarem Kaliumphaspat-Puffer resuspendiert und rezentrifugiert, (das Ganze jeweils 3mal mit 10% des Kulturvolumens). Danach wurde das Zell-Pellet im minimalst möglichen Volumen mit Kaliumphosphat-Puffer resuspendiert und unter Eiskühlung mit Ultraschall (10 mal für je 30 sec.; Pause jeweils 20 sec.) beschallt. Aus diesem Rohextrakt wurden dann die Zelltrümmer mittels Zentrifugation (20 min. bei mindestens 10'000 upm) abgetrennt.

Zur Bildung von 7-Aminocephalosporansäure wurde dieses Rohextrakt (Proteinkonzentration 2 mg/ml) auf Raumtemperatur erwärmt, und dann wurde Malonyl-7-Aminocephalosporansäure, Im Kaliumphosphat-Puffer, in einer Konzentration von 5 mmolar, hinzugegeben. Das Ganze wurde bei 25°C ruhend inkubiert und innerhalb 24 h auf die Bildung von 7-Aminocephalosporansäure analysiert.
Die Analyse erfolgte dünnschichtchromatographisch (Platte: Kieselgel 60 $F^{245}$, Laufmittel: Butanol-Methanol-Eisessig-Wasser in einem Verhältnis von 50:30:3:17). Die Bildung von 7-Aminocephalosporansäure (Rf-Wert: 0,51) ausgehend von Malonyl-7-Aminocephalosporansäure Rf-Wert: 0,43) konnte entweder bei einer UV-Wellenlänge von 254 nm oder nach Besprühen mit Fluram-Reagens (3 mg Fluram; Fluka CH-9470 Buchs in 10 ml Aceton) bei einer UV-Wellenlänge von 366 nm nachgewiesen werden.
Noch ca. 14 h konnten mit diesem Rohextrakt, enthaltend als Enzym eine Malonyl-7-Aminocephlosporansäure-Acylase, 50 bis 100 µ mole 7-Aminocephalosporansäure nachgewiesen werden.

Tabelle 1

a) Mineralsalzmedium Stocklösungen

Stocklösung 1:

| | |
|---|---|
| $NaH_2PO_4 \cdot 2H_2O$ | 156,0 g |
| $NH_4Cl$ | 10,0 g |
| $K_2SO_4$ | 1,2 g |
| | ---------- |
| Aqua dest. | 500,0 ml |

Stocklösung 2:

| | |
|---|---|
| p-Aminobenzoesäure | 8,0 mg |
| D-Biotin | 2,0 mg |
| Nikotinsäure | 20,0 mg |
| Ca-D-Panthothenat | 10,0 mg |
| Pyridoxalhydrochlorid | 30,0 mg |
| Thiamindichlorid | 20,0 mg |
| Cyanocobalamin | 10,0 mg |
| | ---------- |
| Aqua dest. | 100,0 ml sterilfiltrieren |

Stocklösung 3:

| | | |
|---|---|---|
| HCl (37%) | 7,0 | ml |
| $FeCl_2 \cdot 4H_2O$ | 1,5 | g |
| $ZnCl_2$ | 0,07 | g |
| $MnCl_2 \cdot 4H_2O$ | 0,1 | g |

| | | |
|---|---|---|
| $H_3BO_3$ | 0,006 | g |
| $CoCl_2 \cdot 6H_2O$ | 0,19 | g |
| $CuCl_2 \cdot 7H_2O$ | 0,002 | g |
| $NiCl_2 \cdot 6H_2O$ | 0,024 | g |
| $Na_2MoO_4 \cdot 2H_2O$ | 0,036 | g |
| Aqua dest. | 1000,0 | ml, autoklavieren, 121°C, 20 min |

Stocklösung 4:

| | | |
|---|---|---|
| NaOH | 0,5 | g |
| $Na_2SeO_3 \cdot 5H_2O$ | 0,003 | g |
| $Na_2WO_4 \cdot 2H_2O$ | 0,004 | g |
| Aqua dest. | 1000,0 | ml, autoklavieren, 121°C, 20 min |

Stocklösung 5:

| | | |
|---|---|---|
| $MgCl_2 \cdot 6H_2O$ | 40,0 | g |
| $CaCl_2 \cdot 2H_2O$ | 5,0 | g |
| Aqua dest. | 200,0 | ml, autoklavieren, 121°C, 20 min |

Stocklösung 6:

| | | |
|---|---|---|
| Desacetylcephalosporin C-Lacton | 3,55 | g |
| Aqua dest. | 60,0 | ml |
| Penicillinase (EC 3.5.2.6.) (Sigma p0389) | 1,0 | mg (25'000 Units) |
| pH mit NaOH auf | 7,0 | eingestellt |
| mit Aqua dest. auf | 100,0 | ml auffüllen |

b) Herstellung des Mineralsalzmediums

| | | |
|---|---|---|
| Stocklösung 1: | 25,0 | ml |
| pH mit KOH auf | 7,0 | eingesellt, dann mit Aqua dest. auf 950,0 ml auffüllen, autoklavieren, 121°C, 20 min. |

Nach dem Sterilisieren Zugabe von:

| | |
|---|---|
| Stocklösung 2 | 0,5 ml |
| Stocklösung 3 | 1,0 ml |
| Stocklösung 4 | 1,0 ml |
| Stocklösung 5 | 0,5 ml |
| Lösung erhalten aus Beispiel 5 | 50,0 ml |

**Patentansprüche**

**1.** Mikroorganismen, die derart selektioniert sind, dass sie befähigt sind, das Lacton der Formel

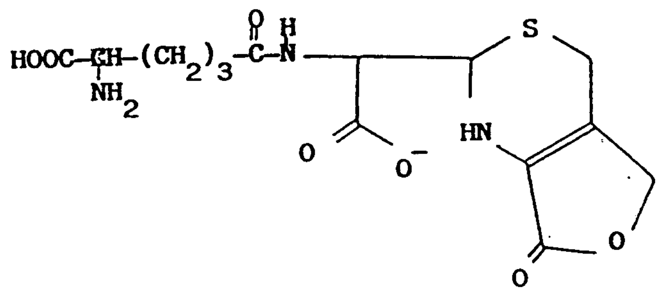

I

oder dessen lösliche Salze
als einzige Kohlenstoff-, Stickstoff- und Energiequelle über das Malonyl-Lacton der Formel

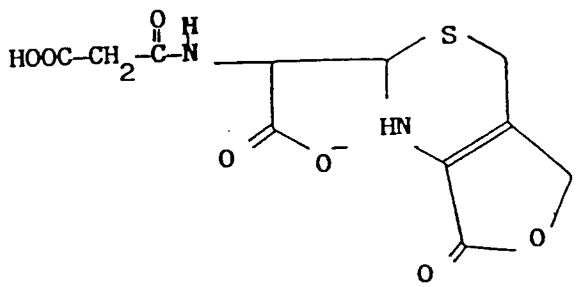

II

oder dessen lösliche Salze
zu verwerten und letztere nicht zu katabolisieren.

2. Mikroorganismen nach Patentanspruch 1 mit der Bezeichnung Sphingomonas sp. FB1 (DSM 7007) sowie deren Deszendenten und Mutanten.

3. Mikrobiologisches Verfahren zur Herstellung von Malonyl-7-Aminocephalosporansäure-Derivaten der allgemeinen Formel

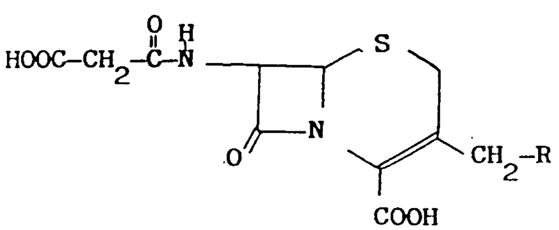

III

oder deren lösliche Salze
worin R ein Wasserstoffatom, eine Hydroxylgruppe oder eine Acetoxygruppe bedeutet, dadurch gekennzeichnet, dass man Cephalosporin C-Derivate der allgemeinen Formel

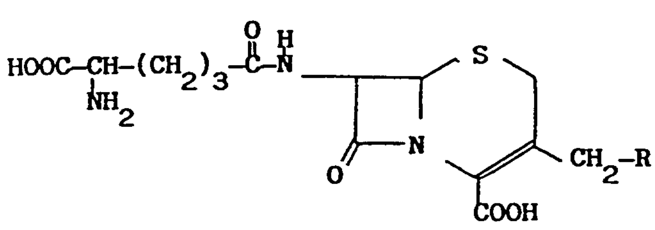

IV

oder deren lösliche Salze, worin R die genannte Bedeutung hat, als Substrat, mittels den Mikroorganismen gemäss Patentanspruch 1 in das Produkt gemäss Formel III überführt.

4. Verfahren nach Patentanspruch 3, dadurch gekennzeichnet, dass man die Umsetzung mittels den Mikroorganismen Sphingomonas sp. FB1 (DSM 7007) oder deren Deszendenten und Mutanten durchführt.

5. Verfahren nach mindestens einem der Patentansprüche 3 und 4, dadurch gekennzeichnet, dass man als Substrat Cephalosporin C anwendet.

6. Verfahren nach mindestens einem der Patentansprüche 3 bis 5, dadurch gekennzeichnet, dass die Umsetzung unter einmaliger oder kontinuierlicher Substratzugabe erfolgt, so dass die Substratkonzentration im Kulturmedium 20 Gew.% nicht übersteigt.

7. Verfahren noch mindestens einem der Patentansprüche 3 bis 6, dadurch gekennzeichnet, dass man die Umsetzung bei einer Temperatur von 0 bis 60 ° C und bei einem pH von 5 bis 9 durchführt.

8. Malonyl-7-Aminocephalosporansäure-Derivate oder deren lösliche Salze der allgemeinen Formel

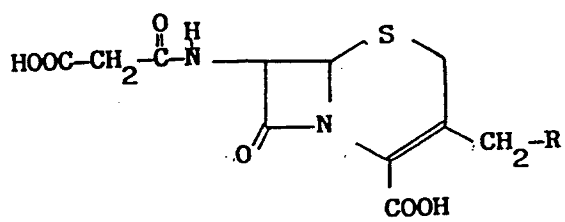

III

worin R ein Wasserstoffatom, eine Hydroxylgruppe oder eine Acetoxygruppe bedeutet.

9. Malonyl-7-Aminocephalosporansäure

10. Mikrobiologisches Verfahren zur Herstellung von 7-Aminocephalosporansäure-Derivaten oder deren lösliche Salze der allgemeinen Formel

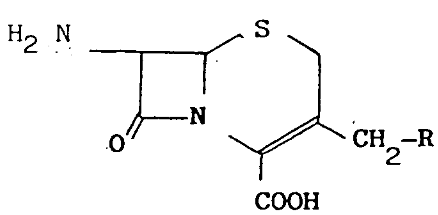

V

worin R die in Patentanspruch 3 genannte Bedeutung hat, dadurch gekennzeichnet, dass man Malonyl-7-Aminocephalosporansäure-Derivate oder deren lösliche Salze der allgemeinen Formel III als Substrat, mittels Mikroorganismen der Gattung Pseudomonas sp. (DSM 7509) oder deren Deszendenten und Mutanten, oder mit zellfreien Enzymen aus diesen Mikroorganismen, in das Produkt gemäss Formel V überführt.

11. Verfahren nach Patentanspruch 10, dadurch gekennzeichnet, dass man als Substrat Malonyl-7-Aminocephalosporansäure anwendet.

12. Verfahren nach mindestens einem der Patentansprüche 10 und 11, dadurch gekennzeichnet, dass die Umsetzung unter einmaliger oder kontinuierlicher Substratzugabe erfolgt, so dass die Substratkonzentration im Kulturmedium 10 Gew.% nicht übersteigt.

**13.** Verfahren nach mindestens einem der Patentansprüche 10 bis 12, dadurch gekennzeichnet, dass man die Umsetzung bei einer Temperatur von 4 bis 50 ° C und bei einem pH von 4 bis 9 durchführt.

**14.** Verwendung von Mikroorganismen der Gattung Pseudomonas sp. (DSM 7509) zur Hydrolyse von Malonyl-7-Aminocephalosporansäure-Derivaten oder deren lösliche Salze der allgemeinen Formel III in 7-Aminocephalosporansäure-Derivate der allgemeinen Formel V.

**15.** Verwendung nach Patentanspruch 14, dadurch gekennzeichnet, dass man Malonyl-7-Aminocephalosporansäure in 7-Aminocephalosporansäure hydrolysiert.